Europäisches Patentamt

European Patent Office

Office européen des brevets

(19)

(11) Publication number: **0 234 897**
**A2**

# EUROPEAN PATENT APPLICATION

(12)

(21) Application number: 87301528.3

(22) Date of filing: 23.02.87

(51) Int. Cl.⁴: **A 61 K 31/57**
**A 61 K 9/50, A 61 K 47/00**

(30) Priority: 24.02.86 US 832297

(43) Date of publication of application:
02.09.87 Bulletin 87/36

(84) Designated Contracting States: DE FR GB IT

(71) Applicant: ETHICON INC.
U.S. Route 22
Somerville New Jersey 08876 (US)

(72) Inventor: Dizerega, Gere Stodder
420 San Juan Place
Pasadena California (US)

Sheffield, Warren Douglas
RD 3, Box 67 Blossom Hill Road
Lebanon, N. J. (US)

(74) Representative: Jones, Alan John et al
CARPMAELS & RANSFORD 43 Bloomsbury Square
London, WC1A 2RA (GB)

(54) Inhibition of post-surgical adhesion formation by the continual topical administration of glucocorticoid.

(57) Postsurgical adhesions are reduced or prevented by the topical administration to the site of surgical trauma of tissue a glucocorticoid, in small amounts continually over the critical wound healing period.

EP 0 234 897 A2

Bundesdruckerei Berlin

**Description**

INHIBITION OF POST-SURGICAL ADHESION FORMATION BY THE CONTINUAL TOPICAL ADMINISTRATION
OF GLUCOCORTICOID

The invention relates to the inhibition of postsurgical adhesions formation.

Adhesion formation is a major post-surgical complication with no practial solution. The incidence of adhesion formation following surgery approaches 100 per cent, according to some sources, with a clinically significant complication rate of about 5 to 10 per cent, depending on the type of surgery. Among such complications are bowel obstruction, infertility, and pain. Occasionally, adhesions necessitate a second operative procedure to remove the adhesion, which may in turn further aggravate the problem.

Because of the seriousness of the problem, much medical research has been performed in efforts to find ways to combat adhesions. See, for instance, Stangel et al., "Formation and Prevention of Postoperative Abdominal Adhesions", the Journal of Reproductive Medicine, Vol. 29, No. 3, March 1984 (pages 143-156), and diZerega, "The Cause and Prevention of Postsurgical Adhesions", published by Pregnancy Research Branch, National Institute of Child Health and Human Development, National Instititutes of Health, Building 18, Room 101, Bethesda, MD 20205.

Among the approaches that have been tried for preventing post-surgical adhesion are the following:

Systemic administration of ibuprofen (e.g., see Singer, U.S. Patent No. 4,346,108);

Parenteral administration of antihistamines, corticosteroids, and antibiotics;

Intraperitoneal administration of dextran solution and of polyvinylpyrrolidone solution; and

Systemic administration of oxyphenbutazone, a non-steroidal anti-inflammatory drug that acts by inhibiting prostaglandin production.

Glucocorticoids have been administered intraperitoneally as well as systemically in efforts to prevent adhesions. (See the Stengel et al,. article, cited above, on page 147, as well as the articles cited therein.) Some studies have questioned the efficacy of glucocorticoids in adhesion prevention. In high doses, these materials may suppress the immune system and interfere with wound healing.

The present invention provides a pharmaceutical composition for continued topical application comprising a topically effective amount of a glucocorticoid or a pharmaceutically acceptable salt thereof as the active ingredient in a topically applicable pharmaceutically acceptable carrier.

The present invention also provides the use of a glucocorticoid or a pharmaceutically acceptable salt thereof in the production of a topically applicable medicament for continued topical use in inhibiting the formation of post-surgical adhesions in mammals.

In use, the glucocorticoid is applied topically to the site of surgical trauma continually the critically wound healing period, e.g. from two to seven days, post-operatively. The following articles have reported various modes of administration of glucocorticoids to combat post-surgical adhesions:

Seitz et al., Fertility and Sterility, Vol. 24, No. 12, pages 935-940 (December 1973);

Shikata et al., World J. Surg., Vol. 1, No. 3, pages 389-395 (May, 1977);

Glucksman et al., Surgery, Volume 60, No. 2, Pages 352-360 (August, 1966);

Pfeffer et al., Fertility and Sterility, Vol. 34, No. 2, pages 162-168 (August, 1980);

Stangel et al., The Journal of Reproductive Medicine, Vol. 29, No. 3, pages 143-156 (March, 1984);

Pfeffer, Fertility and Sterility, Vol. 33, No. 3, March, 1980, pages 245-256 (especially pages 249-250); and

Eskeland, Acta. Chir. Scand., Vol. 125, pages 91-106 (1963).

Among the modes of administration that have been reported are systemic, intraperitoneal (usually in a single dose just after the surgical procedure, although one article reported a second intraperitoneal dose one week after the procedure), or a combination of the two. Several of the articles have questioned the advisability of using glucocorticoids to combat post-surgical adhesion because of the side affects, including immuno-suppressant effects, infection, and interference with wound healing. In a few cases, glucocorticoids were found to be ineffective in combatting post-surgical adhesions.

The pharmacologically active compositions that are employed in the invention are the glucocorticoids, such as dexamethasone, prednisone, prednisolone, hydrocortisone, and the like.

The glucocorticoids are a well known class of materials. Their nature and production are well known. Many are commercially available.

In use, the active agent is applied topically continually to the site of surgical trauma in small effective amounts for at least two and up to about seven days after the operation, to thereby eliminate or substantially reduce the incidence of post-surgical adhesions. Preferably, the administration of the active agent is effected by administering the active agent topically to the site of surgical trauma in a controlled release carrier. Carriers for the active agent include lipids, such as phospholipid micelles or vesicles, dextran, polymers (especially p-dioxanone, lactide, and/or glycolide based absorbable polymers), most preferably in the form of microcapsules or other controlled release carriers that are capable of releasing the active drug for a period of at least two and up to about seven days.

One procedure for preparing a polymeric microcapsule containing a drug is the following:

1. The drug and the polymer are dissolved in a volatile organic solvent such as methylene chloride or diethyl ether;

2. The solvent containing the drug and polymer is dispersed in water with a dispersing a agent such as methylene chloride or diethyl ether;

3. The organic solvent is evaporated from the dispersion product of step 2, either by mild heating or by vacuum evaporation, or by a combination of the two; and

4. The resulting microcapsules are recovered from the aqueous dispersion by customary procedures such as filtration, centrifugation, or the like, usually coupled with one or more washing steps.

A glucocorticoid can be encapsulated in a polymeric microcapsule by procedures analogous to known procedures.

Methods for encapsulating drugs in lipid carriers are known in the art. For instance, one procedure for encapsulating a drug in a phospholipid vesicle is the following:

a lipid or mixture of lipids such as lecithin or other phospholipid is dissolved in an organic solvent such as diethyl ether; and

an aqueous phase containing the material to be encapsulated is added to the lipid solution, and the mixture is agitated as by exposing it to ultrasonic sound waves (sonicated). Preferably, the organic solvent is removed during sonication, as by use of heat or vacuum or both, although in some cases the solvent can be removed after the sonication.

The following United States patents describe the preparation, by various procedures, of phospholipid vesicles containing various medicaments:

Lenk et al. No. 4,522,803
Baldeschwieler et at. No. 4,310,505
Rahman et al. No. 3,993,754
Mezei et al. No. 4,485,054
Gersonde et al. No. 4,452,747
Ash et al. No. 4,448,765
Kelly No. 4,356,167
Papahadjopoulos et al. No. 4,241,046
Suzuki et al. No. 4,016,100
Steck et al. No. 4,186,183
Sache et al. No. 4,239,754

See also Callahan et al., European Patent Application No. 0126580, published November 28, 1984, and Gregoriadis, "The Carrier Potential of Liposomes In Biology and Medicine", New England Journal of Medicine, Vol. 295, pp. 704-710 and pp. 765-770 (Sept. 23 and 30, 1976).

The foregoing are incorporated herein by reference.

Other procedures for incorporating drugs in phospholipids (micelles or liposomes) are described in Sears, U.S. Patent Nos. 4,426,330 and 4,145,410, and Sears et al., U.S. Patent No. 4,298,594, the disclosures of which are incorporated herein by reference.

Glucocorticoids can be encapsulated in a phospholipid vesicle or micelle by procedures analogous to those described in the foregoing references.

The glucocorticoid is administered topically to the site of surgical trauma in small effective quantities continually over the critical wound healing period (which period varies from patient to patient and with the type of surgical trauma encountered, but is usually from about two to five days and in some cases up to seven days or more, post-operatively, beginning before wound healing starts, i.e., just after the surgical procedure).

The examples below illustrate the order of magnitude of effective quantities of the drug and the period of time over which the drug is preferably administered for effective results.

The following experiments use rabbit models to illustrate the adhesion prevention effectiveness of the continual topical administration of dexamethasone in small amounts to the site of surgical trauma:

Example 1

New Zealand white female rabbits (1.8-2.0 kg - see U.S. Patent No. 3,995,631) underwent midline laparotomy using acelepromazine and ketamine anaesthesia. A 3x5 centimeter abrasion was produced over the right-lateral peritoneal side-wall by scraping the surface peritoneum with a scalpel until punctate bleeding developed over the entire 3x5 centimeter area. A second abrasion covering the same total area (15 cm$^2$) using the same technique was developed 1.5-2.0 centimeters inferior to the initial site along the right-lateral peritoneal side-wall. This second site was used as a vehicle control.

A minature osmotic pump (Alzet mini pump, model 2M1L, see US-A-3 995 631) was used to deliver the anti-adhesion agent continuously in small doses to the site of the surgical trauma in the test rabbits over a period of time, up to seven days.

A pump was sewn into the right dorsal subcutaneous space of the test rabbit with VICRYL (Polyglactin 910) sutures placed 3 to 5 millimeters from each end of the pump. A catheter leading from the pump into the peritoneal cavity of each rabbit was placed 2 to 3 millimeters over the injury test site. The catheter was secured in place by two 3/0 VICRYL sutures which did not involve the site of the injury. A similar pump and catheter system containing vehicle only was implanted in the middle portion of the inferior (control) abrasion.

The mini pump had a pumping rate of 0.5 microliter per hour. Each pump contained two milliliters of liquid, either 0.1 M phosphate buffered saline ("PBS") (pH = 6.5) plus the indicated amount of dexamethasone or PBS alone, for the vehicle controls.

Seven days after the day of abrasion, the rabbits were sacrificed by pentobarbital overdose. The extent of

3

adhesions was evaluated as follows:
1. No adhesions
2. Filmy adhesions (separable)
3. Mild adhesions (not separable)
4. Moderate adhesions (not separable)
5. Severe adhesions (not separable)

In each series of experiments, five rabbits were used. In the treatment sites, the amount of dexamethasone was varied from series to series. The amounts used were 24, 4.8, 0.98, 0.192, 0.0384, and 0.00768 mg/ml. The results of these experiments are displayed below in Tables I through VI:

## Table I

### 24 mg/ml

| Rabbit No. | Treatment | Vehicle Control | Bleeding |
|---|---|---|---|
| 1 | 3 | 3 | yes |
| 2 | 1 | 5 | yes |
| 3 | 1 | 1 | no |
| 4 | 1 | 3 | no |
| 5 | 1 | 4 | no |

## Table II

### 4.8 mg/ml

| Rabbit No. | Treatment | Vehicle Control | Bleeding |
|---|---|---|---|
| 1 | 1 | 3 | yes |
| 2 | 1 | 5 | no |
| 3 | 1 | 5 | no |
| 4 | 1 | 5 | no |
| 5 | 4 | 1 | yes |

## Table III
### 0.98 mg/ml

| Rabbit No. | Treatment | Vehicle Control | Bleeding |
|---|---|---|---|
| 1 | 1 | 3 | yes |
| 2 | 1 | 3 | yes |
| 3 | 1 | 5 | yes |
| 4 | 3 | 5 | yes |
| 5 | 1 | 1 | yes |

## Table IV
### 0.192 mg/ml

| Rabbit No. | Treatment | Vehicle Control | Bleeding |
|---|---|---|---|
| 1 | 1 | 4 | no |
| 2 | 3 | 4 | no |
| 3 | 1 | 4 | no |
| 4 | 1 | 5 | no |
| 5 | 3 | 5 | no |

## Table V
### 0.0384 mg/ml

| Rabbit No. | Treatment | Vehicle Control | Bleeding |
|---|---|---|---|
| 1 | 1 | 5 | no |
| 2 | 3 | 1 | no |
| 3 | 4 | 4 | no |
| 4 | 1 | 1 | no |
| 5 | 4 | 4 | yes |

## Table VI

### 0.00768 mg/ml

| Rabbit No. | Treatment | Vehicle Control | Bleeding |
|---|---|---|---|
| 1 | 1 | 1 | no |
| 2 | 4 | 4 | no |
| 3 | 3 | 5 | no |
| 4 | 5 | 4 | no |
| 5 | 4 | 5 | no |

The quantities of dexamethasone delivered to the site of surgical trauma in the foregoing experiments are calculated as follows (using the 24 mg/ml series as an illustrative example):

$0.5 \times 10^{-6}$ liter/hour delivered of solution containing 24 mg of dexamethasone per $10^{-3}$ liter, equals an hourly rate of $0.5 \times 10^{-6} \times 24/10^{-3}$ or $1.2 \times 10^{-2}$ mg/hr. Expressed in terms of mg/kg/day, since the average rabbit weighed about 2 kilograms, the dosage was $1.2 \times 10^{-2} \times 24/2$, or $1.44 \times 10^{-1}$ mg/kg/day.

Table VII displays the dosages administered in the foregoing six series of experiments:

## Table VII

### Dosage, weight/time basis

| Series | mg/ml | mg/hr | mg/kg/day |
|---|---|---|---|
| I | 24 | $1.2 \times 10^{-2}$ | $1.44 \times 10^{-1}$ |
| II | 4.8 | $2.4 \times 10^{-3}$ | $2.88 \times 10^{-2}$ |
| III | 0.98 | $0.49 \times 10^{-3}$ | $5.88 \times 10^{-3}$ |
| IV | 0.192 | $0.96 \times 10^{-4}$ | $11.5 \times 10^{-4}$ |
| V | 0.0384 | $0.192 \times 10^{-4}$ | $2.3 \times 10^{-4}$ |
| VI | 0.00768 | $0.384 \times 10^{-5}$ | $4.6 \times 10^{-5}$ |

The experiments reported above indicate that in the rabbit model the threshold dosage above which significant beneficial anti-adhesion effects are obtained with dexamethasone is between 0.192 and $0.96 \times 10^{-4}$ mg/hr, or between 2.3 and $11.5 \times 10^{-4}$ mg/kg/day. A reasonable estimate of the threshold dosage is about $5 \times 10^{-4}$ mg/kg/day in this model.

In the larger dosages, bleeding was an undesirable side effect in a significant proportion of cases. The dosage below which bleeding ceased to be a significant side effect was between $11.5 \times 10^{-4}$ and $5.88 \times 10^{-3}$ mg/kg/day. A reasonable estimate of the dosage in this model below which bleeding would not be expected to be a significant side effect is about $3.5 \times 10^{-3}$ mg/mg/day.

The area of traumatized tissue may be a relevant factor in determining the amount of medication to administer. In the rabbit experiments reported above, the area of deliberately traumatized tissue was about 15 $cm^2$. Thus, the dosage rates discussed above could be further limited by also expressing them in terms of the surface area of tissue to be treated. Expressed in these terms, the threshold dosage was about $1 \times 10^{-3}$ mg/day/15 $cm^2$, or about $0.67 \times 10^{-4}$ mg/day/$cm^2$, and the dosage below which bleeding would not be expected to be a significant side effect was about $7 \times 10^{-3}$ mg/day/15 $cm^2$, or about $0.47 \times 10^{-3}$ mg/day/$cm^2$.

The active ingredient is administered to the site of surgical trauma topically. Such topical administration can be by spraying, lavage, dripping on the site, by catheter administration, or the like. The exact method of administration chosen is not critical, as long as an effective dose is administered continually over the critical wound healing period, which is usually from two to seven days, post-operatively, beginning just after the operation (i.e., before wound healing has begun).

**Claims**

1. A pharmaceutical composition for continual topical application comprising a topically effective amount of a glucocorticoid or a pharmaceutically acceptable salt thereof as the active ingredient in a topically applicable pharmaceutically acceptable carrier.

2. The composition of claim 1, wherein the carrier is a controlled release carrier which releases the active ingredient in an effective amount over a period of time.

3. The composition of claim 2 wherein said period of time is from two to seven days.

4. The composition of claim 2 or claim 3 wherein said carrier is a phospholipid.

5. The composition of claim 4 wherein said phospholipid is a phospholipid vesicle.

6. The composition of claim 2 or claim 3 wherein said carrier is an absorbable polymer.

7. The composition of any one of claims 1 to 6 which is adpated to be administered at a rate of at least $5 \times 10^{-4}$ mg/kg/day of active ingredient.

8. The composition of any one of claims 1 to 6 which is adapted to be administered at a rate of at least $0.67 \times 10^{-4}$ mg/day/$cm^2$ of surgical trauma.

9. Use of a glucocorticoid or a pharmaceutically acceptable salt thereof in the production of a topically applicable medicament for continual topical use in inhibiting the formation of post-surgical adhesions in mammals.

10. Use of a glucocorticoid or a pharmaceutically acceptable salt thereof in the production of a composition according to any of claims 1 to 8 for continual topical use in inhibiting the formation of post-surgical adhesions in mammals.